# EUROPEAN PATENT APPLICATION

(11) **EP 3 037 195 A1**
(43) Date of publication of application: **29.06.2016**
(21) Application number: 13891840.4
(22) Date of filing: 28.08.2013
(51) Int. Cl.: B22F 9/24, G01N 21/64, C09K 11/88, A61P 35/00

(54) **GOLD NANOFLOWER STRUCTURE AND GOLD NANOFLOWER/QUANTUM DOT COMPOSITE PROBE FOR LIVING CELL IMMUNOFLUORESCENT LABELING AND PHOTOTHERMAL THERAPY**

(30) Priority: 21.08.2013 CN 201310365333
(71) Applicant: The First Affiliated Hospital of Anhui Medical University, Hefei City, Anhui 230022 (CN)
(72) Inventor: ZHU, Lixin, Hefei City Anhui 230022 (CN); JIANG, Tongtong, Hefei City Anhui 230022 (CN); XU, Xiaoliang, Hefei City Anhui 230022 (CN)
(74) Representative: LEITZINGER OY
(86) International application number: PCT/CN2013/082425
(87) International publication number: WO 2015/024274

(57) **Abstract**

The present invention provides are a gold nanoflower structure and a preparation method therefor. The gold nanoflower structure is a gold nanoflower particle, with round-head columns being uniformly distributed at the periphery thereof, obtained by using gold octahedrons, gold balls or gold tetrahedrons as seed crystals and reducing chloroauric acid by using weak reductant in an environment of high-concentration polyvinylpyrrolidone. In addition, also provided in the present invention are a gold nanoflower/quantum dot composite probe for living cell immunofluorescent labeling and photothermal therapy, a preparation method therefor and a use thereof. In comparison with traditional probes, the probe, incorporates the features of photothermal therapy and fluorescent labeling, and is capable of killing cancer cells in an effective and directional way. Two light sources are adopted to bring a tremendous photothermal conversion efficiency and a greater enhancement on fluorescence intensity of quantum dots respectively, thus mutual interference of two effects are avoided tactfully. The coating of silicon dioxide averts the biotoxicity of the gold nanoflower and the quantum dot effectually, enabling the surface of the composite probe to be easily functionalized and also imparting an extraordinarily excellent biocompatibility to the composite probe.

## Description

### Technical Field

The present invention is related to the technical field of a composite nano-probe, and specifically related to a gold nanoflower/quantum dot composite probe for living cell immunofluorescent labeling and photothermal therapy.

### Background

A quantum dot (QD) is a nano-material that artificially synthesized from a small amount of atoms, and also be called "artificial atom", which emits fluorescence after excited. The excitation and emission wavelength of the fluorescence from the QD can be adjusted via changing the structure of QD. After being bonded with a specific antibody and used for labeling and tracing the target cells, the physical/chemical and luminescence stability of the quantum dots (QDs) can be held in cells for a long term. QDs can be used to replace the traditional dyes as the fluorescent labeler, to overcome the shortcomings of the dyes including the large molecule (labeling cannot be used in living cells for the cells that need to be punched), the poor photochemical instability and optical property. Therefore the QDs can be used for the immunofluorescent labeling of the living cells, wherein the near infrared (NIR) QDs should be suitable for the labeling in vivo, where only small amount of light in the NIR range can be absorbed by the tissues.

A photothermal therapy is a treatment method, using a nano-metal structured photothermal material to absorb the light in NIR range (which is an optical window for the blood and soft tissues of animals or human being, and the light in NIR range could transmit to the deep tissues through human skin) with the mechanism of surface plasmon resonance (SPR) enhanced electrons group strike on the metalic bonds, to generate the phonons (i.e., heat energy), results in a partial high temperature and thus making the cancer cells apoptosis. Researches show that the nanostructure of the photothermal material is directly related to a photothermal conversion threshold (generally calculated by Watt per square centimetre, i.e., W/cm²) and the temperature. The lower the threshold and the faster the temperature increase, the more effective for stopping the activity of cancer cells, and the less damage to the normal organism. During last 10 years, the metallic photothermal material has been undergoing a variety of gold nanostructures, such as nano-ball (conversion threshold of 200 W/cm²), nano-rod (160 W/cm²), nano-shell (10 W/cm²), nano-cage (2 W/cm²), and nano-star (1.8W/cm²).

Due to its larger photo absorption photothermal conversion section based on the local SPR area, the gold nanoparticle has become a good material for heat production. A fluorescent QD has a broader excitation band and a narrower emission band, which makes it a good material for tracing in vivo. Combining the two excellent properties to prepare a dual-functional photothermal/fluorescence composite nano probe, so as to make it possible to realize the integration of the treatment and diagnosing modes.

With a larger absorption section, a normal photothermal structured metallic material has a weaker optical enhancement effect, which easily results in weakening the fluorescence from the QDs nearby. Such as the patent No. 201310117034.4 entitled "Preparation and applications of a gold nano-star@quantum dots composite cell probe with dual-functions of photothermal and fluorescence enhancement" discloses a composite probe, which possesses dual-functions of photothermal conversion and SPR enhanced fluorescence, in which however, still needs to be improved for being more suitable as a photothermal/fluorescence composite label in vivo, for the following three reasons: (1) a gold nano-star has less numbers of spines, the lengths of which are difficult to be fabricated with a consistent, thus the gold nano-star has a very wide absorption spectrum width that overlaps that of the near-infrared QDs, in this case some photothermal effect may also be generated while the QDs are excited for luminescence (i.e., for fluorescence tracing), resulting in the impact for the brightness of the near-infrared luminescence (the photothermal and the light emission can be simultaneously enhanced when the light wavelength shorter than orange and is only suitable for label shallow tumors near skin; (2) the gold nano-star has a photothermal threshold of about 1.8W/cm², which can be further reduced; (3) due to the longer spines of the gold nano-star, it is difficult to control the final size of a composite probe to be less than 100nm, which may exert some negative impact when it is labeled a living cell.

### Content of invention

The objective of the invention is to provide a gold nanoflower structure and a preparation method, to enhanced fluorescent emission from the QDs, the QDs are for the fluorescence tracing of cancer cells and thus for targeting treatment of the cancer cells through a photothermal conversion function.

Another objective of the invention is to provide a gold nanoflower /quantum dot composite probe for living cell immunofluorescent labeling and photothermal therapy and its preparation method.

First, the present invention prepares a gold nanoflower structure on the improvement to various gold nanostructures. Fabrication process of gold nanoflower structure is: use a gold octahedron, or gold ball or gold tetrahedron as seed, and obtains a gold nano particle with round head column arranged around circumferential side thereof, i.e., the chrysanthemum-like gold nanoflower by reducing chloroauric acid with a weak reductant in a high concentration PVP (polyvinylpyrrolidone) environment.

Preferably, the diameter of the gold nanoflower is 45-150 nm, and the column length is 10-30 nm.

The nanoflower prepared by the present invention is the nano-particle generated by modifying with a surfactant, for the high stability of the particle which coating by the surfactant, the nanoflower can be stored for a long time, and specially a very good stability in water and an ethanol solution.

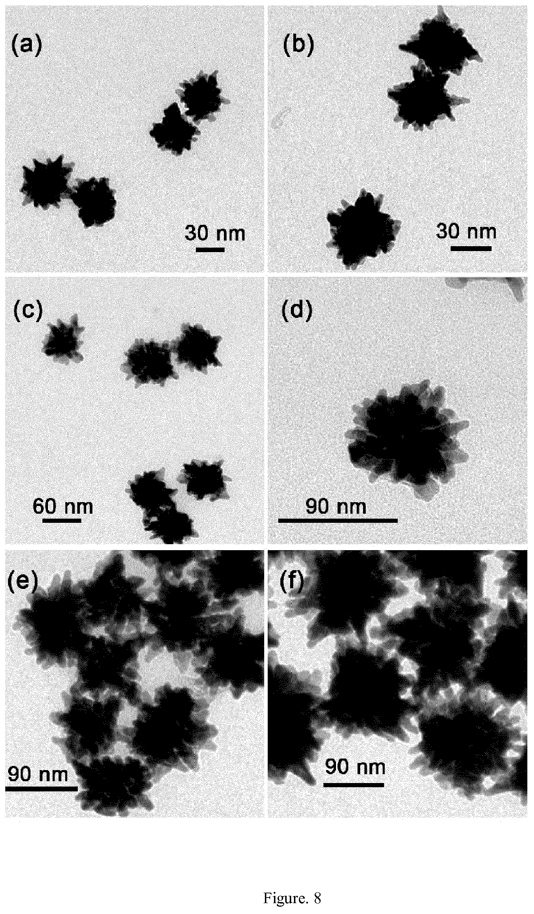

Comparing with spines on a gold nanostar, the nanoflower prepared by the present invention has more columns with comparatively consistent lengths, and thus has following advantages: (1) its absorption spectrum can be controlled narrower and not overlapped with the absorption spectrum of near-infrared QDs, and the brightness of the fluorescence is not attenuated, rather, the emission intensity of the NIR QDs is increased by 35 % as the gold nanoflower possesses fluorescent enhancement effect; (2) the photothermal threshold is further reduced to about 1.2 W/cm²; and (3) for the shorter length of the columns of the nanoflower, when linking QDs and wrapping SiO₂ for preparing a composite probe, the diameter of which can be easily controlled at less than 100 nm, which is suitably used for labeling a cell in vivo. The nanoflower with different sizes prepared in the present invention is shown in Fig. 8.

Generally, in a fluorescent enhancement effect, the control for separating the surface plasmon resonance (SPR) and absorption/emission from the fluorescence materials (i.e. QDs) is deemed as very important. In addition, when a frequency of an incident light and an inherent frequency of a metal nanostructure are coupled, a phenomenon namely local surface plasmon resonance (LSPR) will occur. A resonance excitation will cause a strong local electric field, which may dramatically increase the absorption and excitation rate. The SPR peak of metal particles depends on many factors, including material, size, morphology, etc. The nanoflower structure can creates two SPR peaks, one is between 500 nanometre (nm) to 650 nm, which is well matched the excitation peak of the NIR QDs and can effectively increase fluorescence of the QDs. Another peak can be adjusted to 700 nm to infrared region (such as the commonly used 808 nm) and is helpful for photothermal therapy in vivo. If the probe is used for fluorescence labeling of cell strains in vitro, QDs with different visible light emission peaks (for example, 470 nm blue, 530 nm green, and 580 nm yellow light) can be linked to the cell. The excitation wavelength of these QDs can be a single ultraviolet light uniformly with no decrease in emission (no necessarily to use a designated wavelength to excite the emission from a specific material as the dye).

Further, the present invention provides a method for preparing the gold nanoflower, comprising the following specific steps:
(1) obtaining a gold seed crystals solution by reducing chloroauric acid with NaBH₄, followed by growing a larger gold nano-ball, gold octahedron or gold tetrahedron with an assistant of reductant; and
(2) using the gold nano-ball, gold octahedron or gold tetrahedron obtained in step (1) as seed crystals, growing a gold nanoflower by reducing chloroauric acid with a weak reductant, in an environment of high-concentration PVP; and thus obtaining chrysanthemum-like gold nanoflower with different sizes and column lengths by changing the factors, such as PVP concentration, amount of chloroauric acid and gold seed, and type of the gold seed crystals. Preferably, mixing the solution of the NaBH₄ and chloroauric acid further comprises an aging step, the aging temperature is between 27 and 32 °C and the aging time is more than 3 hours. Preferably, the molar ratio of the NaBH₄ to chloroauric acid in step (1) is 6-24:2-10. Preferably, the reductant in step (1) is ascorbic acid.

Preferably, the environment of high-concentration PVP in step (2) refers to a polyvinylpyrrolidone solution with a concentration of 4-10 mM.

Preferably, the weak reductant in step (2) is N,N-dimethylformamide; the volume ratio of the chloroauric acid to gold seed crystals solution in step (2) is 1:1∼1:30. For instance, in a preferred example of the present invention, the amount of the chloroauric acid is 100-500 µL, 24.28 mM; the amount of the gold seed crystals is 100-3000 µL, 0.1 mM; the amount of N,N-dimethylformamide (DMF) is 10-30 mL, and the amount of PVP (solid powder) is 4-10 mM.

On the other hand, the present invention provides a gold nanoflower /QDs composite probe for living cell immunofluorescent labeling and photothermal therapy, the gold nanoflower/QDs composite probe includes the gold nanoflower structure.

In the present example, the probe comprises, from inside to outside, the gold nanoflower, the silica nano shell layer, the QDs and the silica nano shell layer; includes wrapping a silica layer outside the gold nanoflower through Stöber hydrolysis based on the prepared gold nanoflower to form a silica ball wrapped with the gold nanoflower; then linking the QDs to the surface of the silica ball by modifying the surface of silica, to form a silica ball composite material, with the gold nanoflower wrapped inside and the QDs linked outside; and wrapping the surface of the composite material with another silica nano shell layer at the end.

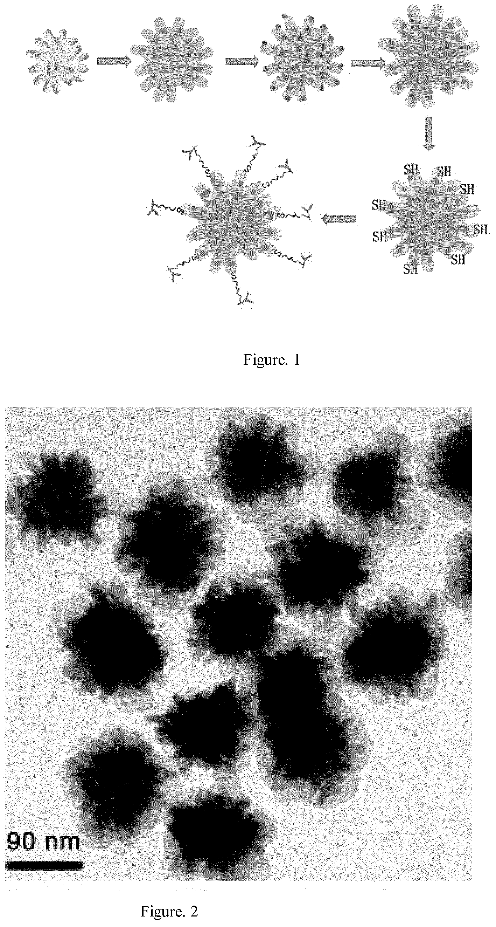

Further, the present invention also provides a process for the preparation of a gold nanoflower /QDs composite probe for living cell immunofluorescent labeling and photothermal therapy, which comprises the following specific steps:
(1) preparing a gold nanoflower;
(2) preparing gold nanoflower@ silica@QDs composite material by:
   centrifugally washing the gold nanoflower obtained in step (1), dissolving the nanoflower in a mixing liquid of ethanol, water and ammonia with ultrasound, followed by dropwise adding an ethanol solution of tetraethyl orthosilicate to the reaction solution for wrapping the gold nanoflower with the silica ball shell, centrifugally washing after reacting for 12 hours, forming a composite structure of gold nanoflower and the silica;
   and surface modifying the above structure, adding aqueous solution of fluorescence QDs with carboxyl to the modified composite structure of gold nanoflower and the silica, reacting for 24 hours, and then centrifugalizing and dispersing in ethyl ethanol, obtaining an ethanol solution of gold nanoflower @silica @quantum dots composite material.
(3) preparing a gold nanoflower@SiO₂@QDS composite probe by:
   adding ammonia to the composite material prepared in step (2) assistant with ultrasound, followed by dropwise adding an aqueous solution of tetraethyl orthosilicate, stirring at room temperature for reacting for 24 hours, centrifugal washing; modifying with various silane coupling agents to bring different modifying groups on surface thereof, and obtaining the composite probe at the end. The specific synthesis procedure chart is shown in Fig 1.

Preferably, the volume concentration of tetraethyl orthosilicate ethanol solution in step (2) is 1 %; the volume ratio of the ethanol, water, tetraethyl orthosilicate ethanol solution, and ammonia is 10-40:2-10: 0.4-1:0.2-1; the volume ratio of the gold nanoflower to the above mixing solution is 0.1-1:5-20.

Preferably, the modifier in step (2) is 3-aminopropyl trimethoxysilane (APTMS) or aminopropyltriethoxysilane; the modifier can be used with a slightly larger amount, the redundant amount can be washed out later by washing, an amount of 200-3000 microlitres can be added when all the particles are prepared.

Preferably, the fluorescence QDs are CdTeS, CdTe, CdSe, Mn-doped ZnS, CdS or CdTe/CdS/ZnS, and the molar number is 0.05-1.0 mM.

Preferably, the silane coupling agent is one of that chosen from vinyl silane coupling agent, amino silane coupling agent, epoxy group silane coupling agent, methacryloxy silane coupling agent, sulfydryl silane coupling agent or carbamido silane coupling agent. The silane coupling agent can be used with a slightly larger amount, the redundant amount can be washed out later by washing, an amount of 150-4000 microlitres, preferably 200-300 microlitres can be added when all the particles are prepared.

Furthermore, the present invention provides a use of the composite probe in biological targeting. Specifically, various biological molecules are linked on various modifying groups on the surface of the composite cell probe for the function of targeting labeling. The biological molecule is preferably an antibody, streptavidin, DNA fragment, biological ligand, and cationic polypeptide, etc. Different antibodies can be linked according to the requirement for labeling different subcell organs in cells. For instance, linking a single antibody CK-19 can specifically trace a micrometastasis of a breast cancer through lymph; and can also label multiple biological indexes for multiple color QDs labeling simultaneously.

The present invention has the following advantages and positive effects:
Compared to a traditional probe, the probe combines photo-thermal therapy and fluorescent labeling together, can target and kill the cancer cells effectively; and uses two light sources for achieving an enormous photo-thermal conversion efficiency and comparatively larger enhancement of the intensity for the QDs fluorescent, separately with avoiding mutual interference of the two effects skillfully. Wrapping with silica can effectively avoid biotoxicity of the gold nanoflower and the QDs, resulting in that the surface of the composite probe can be functionalized easily for having a very good biocompatibility.
   (1) A very high photothermal conversion efficiency can be achieved by matching SPR peak of the gold nanoflower near 800 nm and the 808 nm laser, the photothermal conversion threshold is lower than 1.5 W/cm². Cancer cells can be killed effectively assistant with protecting normal cells from being damaged. Targeting treatment for cancer cells can be realized by linking antibody on the surface of composite nano probe.
   (2) In the composite nano probe, a local electromagnetic field near 600 nm induced by local surface plasmon (LSP) at gold nanoflower is used to enhance a radiation rate of the NIR QDs (630-700nm), thus further enhance the intensity of the emitted fluorescence. Therefore, the cancer cells can be labeled well, and tracing their metastasis in vivo. If being used for fluorescence development of different subcell organs of cell strains in vitro, the QDs with different visible emission (e.g., 470 nm blue light, 530 nm green light, 580 nm yellow light or 620 nm red light) can be linked, these QDs can be excited without any decrease in emission by a ultraviolet source with one wavelength (no necessarily to excite each kind of QDs with different specific wavelength as dyes).
   (3) Compare to the traditional photothermal probe and QDs fluorescence probe, silica is used as barrier such that the probe is not exposed directly in biological bodies or cells, and therefore, the toxicity is shield. And the surface of the silica is functionalized to achieve the function of target labeling.

### Figure captions

The above and/or additional aspects and advantages of the present invention become obvious and understandable easily through the following description about the examples in combination with figures, wherein:
Fig 1. A schematic of synthesis procedure of a gold nanoflower @QDs photothermal fluorescence composite probe.
Fig2. Transmission electron microscopy (TEM) of a gold nanoflower@QDs photothermal fluorescence composite probe of example 4.
Fig 3. Absorption spectrum of the composite probe prepared in examples 1-5, the peak is a suggested excitation wavelength for generating photothermal effect.
Fig 4. (a) Compare the fluorescence of a gold nanoflower@QDs composite probe and the emission spectrum of the same amount of QDs, showing that a luminescence peak is at 650 nm and the luminescence of the composite probe is enhanced by 35 %; (b) Compare the fluorescence of a gold nano-star @QDs composite probe and the emission spectrum of the same amount of QDs, the luminescence peak is at 575 nm and the luminescence of the composite probe is enhanced by 26 %.
Fig 5. Temperature elevating curves (excited power density of 808 nm laser: 1.2 W/cm²) of a composite probe solution prepared in example 4 and the control group (an aqueous solution).
Fig 6. The photothermal curative effect (excite power density of 808 nm laser: 1.2 W/cm²) of the composite probes (linking CK-19 antibody) prepared in example 4 on breast cancer cells (the black small bubbles in the middle of cells are dead cells).
Fig 7. Comparison of the photothermal curative effect (excite power density of 808 nm laser: 1.2 W/cm², and illuminating for 10 minutes) in example 4 for breast cancer cells SK-BR-3 of that (a) without and (b) with linking a composite probe (linking CK-19 antibody).
Fig 8. Several nanoflowers of different sizes obtained by changing amounts of chloroauric acid (CA, 24.28 mM) and the seed crystals. The volume ratios of the CA/seed crystals are (a) 1:16, (b) 3:40, (c) 3:32, (d) 1:8, (e) 3:16, and (f) 3:8. The (a)-(e) correspond to the nanoflower prepared in examples 1-5, respectively.

### Specific Mode for Carrying out the Invention

The following gives detailed description of examples of the present invention, typical examples are given in the figures, wherein the always same or similar symbols represent the same or similar elements, or elements having the same or similar function. The examples described through reference figures given below are for exemplification, which are used only for explanation of the present invention, rather than to be understood as limiting the present invention.

### Chemical medicines:

The following medicines are purchased from Sinopharm Chemical Reagent Co., Ltd.: chloroauric acid (CA, AR), N,N-dimethylformamide (DMF, AR), polyvinyl pyrrolidone (PVP, MW=55000, AR), polyvinyl pyrrolidone (PVP, MW=1000, AR), hexadecyl trimethylammonium bromide (CTAB, AR), hexadecyl trimethyl ammonium chloride (CTAC, AR), ascorbic acid (AA, AR), sodium borohydride (NaBH₄, AR), tetraethyl orthosilicate (TEOS, AR), ammonia (25 % NH₃, AR), and anhydrousethyl ethanol (AR). The preparation methods of 3-trimethoxysilylpropanethiol (MPTMS, sigma, AR), 3-aminopropyl trimethoxysilane (APTMS, sigma, AR), and several different quantum dots (CdTe, CdTe@CdS, CdTeS, etc.) are referred to in the following articles ([1] Sander F. Wuister, Ingmar Swart, Floris van Driel, Stephen G. Hickey, and Celso de Mello Donega, Nano Lett., 3 (2003)504; [2]Hui Peng, Lijuan Zhang, Christian Soeller, and Jadranka Travas-Sejdic, Journal of Luminescence 127 (2007) 721-726; [3] Weiyong Mao, Jiaguo Wu, Li Yang, Changchun Wang, Jia He and Jiyao Chen, Nanotechnology18 (2007) 485611).

### Example 1:

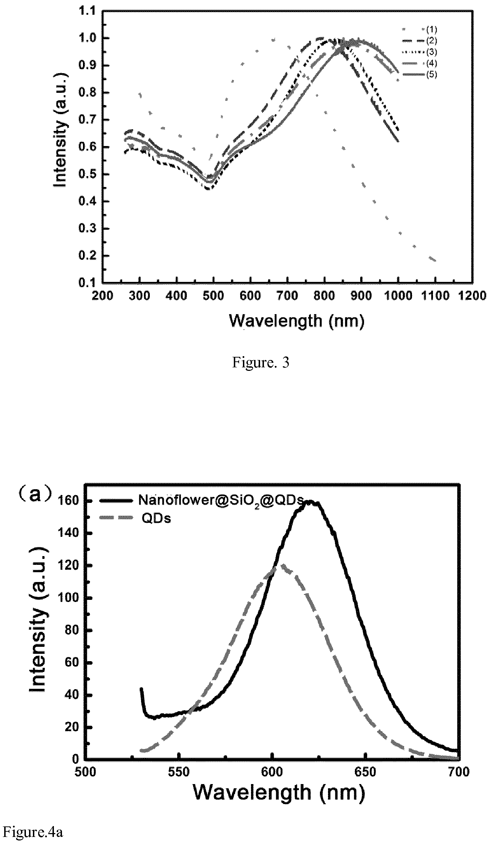

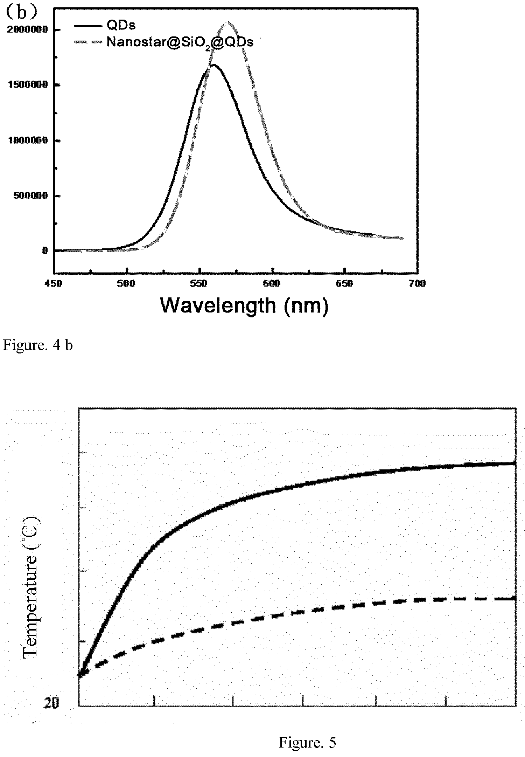

(1) 0.6 mL of 10 mM ice bathed NaBH₄ is added to 10 mL of an aqueous solution that comprising 0.85 mM HAuCl₄ and 0.1M CTAB, the solution is aged for at least 3 hours at the temprature of 29°C. Small gold balls are generated.
(2) 6 mL of 0.5 mM HAuCl₄ solution, 6 mL of 0.2 M CTAC and 4.5 mL of 0.1 M AA are mixed and 0.3 mL of seed crystals prepared in step (1) is added. The solution is centrifugally dissolved in 1 mL of water to obtain larger gold balls.
(3) 5.8 mL DMF (including 0.43 mM PVP (MW=55000)) and 40 µL HAuCl₄ (24.28 mM) are injected into a small bottle, then 200 µL seed crystals prepared in step (2) is added, being stirred at the temprature of 80°C for 1 hour, and the seed crystals for growing gold nanoflower is obtained. The shape and size of the seeds are shown in Fig 8(a).
(4) 15 mL of 9 M PVP DMF solution is added with 150 µL HAuCl₄, and 2400 µL of seed crystals prepared in step (3) is added, being stirred for several hours until the completion of the reaction. The generated gold nanoflower is centrifugally dissolved in an ethanol solution. A figure (as shown in Fig. 3) is used to show a relation of shape and absorption spectrum of the nanoflower obtained in the present invention, indicating that the excitation wavelength is adjusted by the shape. The suggested excitation wavelength for generating a photo-thermal effect can be seen in the first curve from the left in Fig 3, which is 670 nm.
(5) The product of step (4) is added to a mixed solution of 40 mL ethanol, 8 mL water, and 0.8 mL ammonia with ultrasound assisted, then 1.6 mL of 1% TEOS ethanol solution is added, centrifugalizing for 12 hours, to generate a composition of gold nanoflower @silica; then the surface of silica is functionally modified with 200 µL of 3-aminopropyl trimethoxysilane (APTMS), and then 2 mL of CdTeS QDs linked with carboxyl is added. Reacting for 24 hours, and then centrifugally dispersed in ethanol.
(6) Followed by the ethanol solution in step (5) added into the mixed solution of 20 mL ethanol, 4 mL water, and 0.8 mL ammonia, 400 microlitres of 1% TEOS ethanol solution is dropwise added, and reacting for 24 hours; and then 200 µL of MPTMS, APTMS, etc. are used for modifying the surface of the composite structure for having sulfydryl group and amino group on the surface. Then the probes can be transferred to a specified target position. In the present example, the composite probe with a size of 65 nm and the shape is consistent with the TEM of the gold nanoflower @QDs photothermal fluorescence composite probe in Fig 2, the only difference is the wavelength for generating photothermal effect, and being used for different applications (in vivo, in vitro). The temperature elevating curves of the composite probe solution and the control group (an aqueous solution) are about the same as Fig 5, the only difference is the wavelength for generating photothermal effect, and is used for different applications (in vivo, in vitro).

The probe prepared in each example has the same shape of nanoflower wrapped with SiO₂ as shown in Fig 8(a)-(e) (corresponding to examples 1-5), the spectrum corresponding to Fig 3, and is applicable to excitation of a laser device of different wavelength (600-900 nm). Users can make selections according to the required conditions, such as with consideration of universality of an 808 nm laser and its good penetrability in vivo. When one of the absorption peaks of the gold nanoflower is at 808 nm (refer to example 3), a SPR with the emitting wavelength of a laser device is able to be satisfied, resulting in the best photothermal effect. Such analysis of other examples is the same.

### Example 2:

(1) 0.6 mL of 10 mM ice bathed NaBH₄ is added to 10 mL of an aqueous solution that comprising 1.5 mM HAuCl₄ and 0.1M CTAB, the solution is aged for at least 3 hours at the temprature of 29°C. Small gold balls are generated.
(2) 6 mL of 0.5 mM HAuCl₄ solution, 6 mL of 0.2 M CTAC and 4.5 mL of 0.1 M AA are mixed and 0.3 mL of seed crystals prepared in step (1) is added. The solution is centrifugally dissolved in 1 mL of water to obtain larger gold balls.
(3) 5.8 mL DMF (including 0.43 mM PVP (MW=55000)) and 40 µL HAuCl₄ (24.28 mM) are injected into a small bottle, then 200 µL seed crystals prepared in step (2) is added, being stirred at the temprature of 80°C for 1 hour, and the seed crystals for growing gold nanoflower is obtained. The shape and size of the seeds are shown in Fig 8(b).
(4) 15 mL of 9 M PVP DMF solution is added with 150 µL HAuCl₄, and 2400 µL of seed crystals prepared in step (3) is added, being stirred for several hours until the completion of the reaction. The generated gold nanoflower is centrifugally dissolved in an ethanol solution. A figure (as shown in Fig. 3) is used to show a relation of shape and absorption spectrum of the nanoflower obtained in the present invention, indicating that the excitation wavelength is adjusted by the shape. The suggested excitation wavelength for generating a photo-thermal effect can be seen in the second curve from the left in Fig 3, which is 780 nm.
(5) The product of step (4) is added to a mixed solution of 40 mL ethanol, 8 mL water, and 0.8 mL ammonia with ultrasound assisted, then 1.6 mL of 1% TEOS ethanol solution is added, centrifugalizing for 12 hours, to generate a composition of gold nanoflower @silica; then the surface of silica is functionally modified with 200 µL of 3-aminopropyl trimethoxysilane (APTMS), and then 2 mL of CdTeS QDs linked with carboxyl is added. Reacting for 24 hours, and then centrifugally dispersed in ethanol.
(6) Followed by the ethanol solution in step (5) added into the mixed solution of 20 mL ethanol, 4 mL water, and 0.8 mL ammonia, 400 microlitres of 1% TEOS ethanol solution is dropwise added, and reacting for 24 hours; and then 200 µL of MPTMS, APTMS, etc. are used for modifying the surface of the composite structure for having sulfydryl group and amino group on the surface. Then the probes can be transferred to a specified target position. In the present example, the composite probe with a size of 75 nm and the shape is consistent with the TEM of the gold nanoflower @QDs photothermal fluorescence composite probe in Fig 2, the only difference is the wavelength for generating photothermal effect, and being used for different applications (in vivo, in vitro). The temperature elevating curves of the composite probe solution and the control group (an aqueous solution) are about the same as Fig 5, the only difference is the wavelength for generating photothermal effect, and is used for different applications (in vivo, in vitro).

### Example 3:

(1) 0.6 mL of 10 mM ice bathed NaBH₄ is added to 10mL of an aqueous solution that comprising 3 mM HAuCl₄ and 0.1M CTAB, the solution is aged for at least 3 hours at the temprature of 29°C. Small gold balls are generated.
(2) 6 mL of 0.5 mM HAuCl₄ solution, 6 mL of 0.2 M CTAC and 4.5 mL of 0.1 M AA are mixed and 0.3 mL of seed crystals prepared in step (1) is added. The solution is centrifugally dissolved in 1 mL of water to obtain larger gold balls.
(3) 5.8 mL DMF (including 0.43 mM PVP (MW=55000)) and 40 µL HAuCl₄ (24.28 mM) are injected into a small bottle, then 200 µL seed crystals prepared in step (2) is added, being stirred at the temprature of 80°C for 1 hour, and the seed crystals for growing gold nanoflower is obtained. The shape and size of the seeds are shown in Fig 8(c).
(4) 15 mL of 9 M PVP DMF solution is added with 180 µL HAuCl₄, and 1920 µL of seed crystals prepared in step (3) is added, being stirred for several hours until the completion of the reaction. The generated gold nanoflower is centrifugally dissolved in an ethanol solution. The suggested excitation wavelength for generating a photo-thermal effect can be seen in the third curve from the left in Fig 3, which is 808 nm.
(5) The product of step (4) is added to a mixed solution of 40 mL ethanol, 8 mL water, and 0.8 mL ammonia with ultrasound assisted, then 1.6 mL of 1% TEOS ethanol solution is added, centrifugalizing for 12 hours, to generate a composition of gold nanoflower @silica; then the surface of silica is functionally modified with 200 µL of 3-aminopropyl trimethoxysilane (APTMS), and then 2 mL of CdTeS QDs linked with carboxyl is added. Reacting for 24 hours, and then centrifugally dispersed in ethanol.
(6) Followed by the ethanol solution in step (5) added into the mixed solution of 20 mL ethanol, 4 mL water, and 0.8 mL ammonia, 400 microlitres of 1% TEOS ethanol solution is dropwise added, and reacting for 24 hours; and then 200 µL of MPTMS, APTMS, etc. are used for modifying the surface of the composite structure for having sulfydryl group and amino group on the surface. Then the probes can be transferred to a specified target position. In the present example, the composite probe with a size of 85 nm and the shape is consistent with the TEM of the gold nanoflower @QDs photothermal fluorescence composite probe in Fig 2, the only difference is the wavelength for generating photothermal effect, and being used for different applications (in vivo, in vitro). The temperature elevating curves of the composite probe solution and the control group (an aqueous solution) are about the same as Fig 5, the only difference is the wavelength for generating photothermal effect, and is used for different applications (in vivo, in vitro).

### Example 4:

(1) 0.6 mL of 10 mM ice bathed NaBH₄ is added to 10mL of an aqueous solution that comprising 4 mM HAuCl₄ and 0.1M CTAB, the solution is aged for at least 3 hours at the temprature of 29°C. Small gold balls are generated.
(2) 6 mL of 0.5 mM HAuCl₄ solution, 6 mL of 0.2 M CTAC and 4.5 mL of 0.1 M AA are mixed and 0.3 mL of seed crystals prepared in step (1) is added. The solution is centrifugally dissolved in 1 mL of water to obtain larger gold balls.
(3) 5.8 mL DMF (including 0.43 mM PVP (MW=55000)) and 40 µL HAuCl₄ (24.28 mM) are injected into a small bottle, then 200 µL seeds crystals prepared in step (2) is added, being stirred at the temprature of 80°C for 1 hour, and the seed crystals for growing gold nanoflower is obtained. The shape and size of the seeds are shown in Fig 8(d).
(4) 15 mL of 9 M PVP DMF solution is added with 180 µL HAuCl₄, and 1440 µL of seed crystals prepared in step (3) is added, being stirred for several hours until the completion of the reaction. The generated gold nanoflower is centrifugally dissolved in an ethanol solution. The suggested excitation wavelength for generating a photo-thermal effect can be seen in the fourth curve from the left in Fig 3, which is 800-860 nm.
(5) The product of step (4) is added to a mixed solution of 40 mL ethanol, 8 mL water, and 0.8 mL ammonia with ultrasound assisted, then 1.6 mL of 1% TEOS ethanol solution is added, centrifugalizing for 12 hours, to generate a composition of gold nanoflower @silica; then the surface of silica is functionally modified with 200 µL of 3-aminopropyl trimethoxysilane (APTMS), and then 2 mL of CdTeS QDs linked with carboxyl is added. Reacting for 24 hours, and then centrifugally dispersed in ethanol.
(6) Followed by the ethanol solution in step (5) added into the mixed solution of 20 mL ethanol, 4 mL water, and 0.8 mL ammonia, 400 microlitres of 1% TEOS ethanol solution is dropwise added, and reacting for 24 hours; and then 200 µL of MPTMS, APTMS, etc. are used for modifying the surface of the composite structure for having sulfydryl group and amino group on the surface. Then the probes can be transferred to a specified target position. In the present example, the composite probe with a size of 90 nm and the shape is consistent with the TEM of the gold nanoflower @QDs photothermal fluorescence composite probe in Fig 2. The temperature elevating curves of the composite probe solution and the control group (an aqueous solution) are about the same as Fig 5.

Further, comparing the fluorescence of a gold nanoflower @QDs composite probe of the present application and the emitting spectrum of same amount of the QDs, as shown in Fig 4 (a), a luminescence peak of the QDs is at 650 nm, and the intensity is enhanced by 35 %; and as shown in Fig 4 (b), comparing the fluorescence of a gold nano-star @QDs composite probe and a fluorescence spectrum of the same amount of QDs, the luminescence peak of the quantum dot is at 575 nm, and the intensity is enhanced by 26 %. As the quantum dot is wrapped by SiO₂ in the composite probe, red shift occurs in the enhanced luminescence peak. The enhanced luminescence of gold nanoflower @QDs composite probe is in near infrared region, which is more suitable for labeling of a cell in vivo.

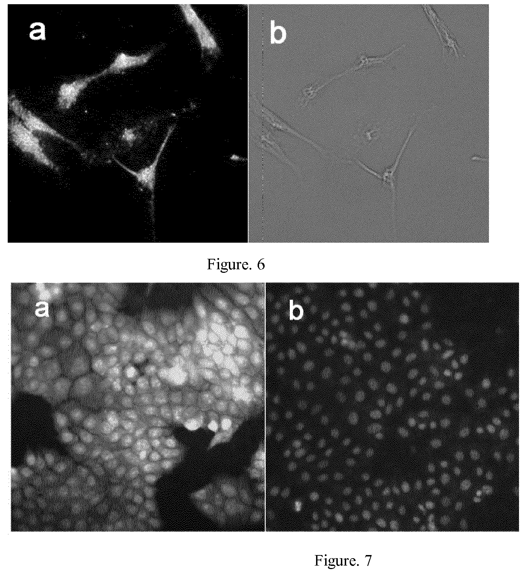

Furthermore, the probe prepared in the present example links to CK-19 antibody by a conventional method, and the photothermal effect to breast cancer cells is detected with a conventional method. The result is shown in Fig 6. The composite probe (linking CK-19 antibody) of the present example has the photothermal effect to breast cancer cells (the black bubbles in the middle of cells are dead cells) (excited power density of 808 nm laser: 1.2 W/cm²). Probes prepared by nano flower of different sizes prepared in other examples have about the same shapes and effect, but the wavelength for generating photothermal effect is different, and being used for different applications.

Further, comparing the photothermal effect of breast cancer cells SK-BR-3 (a) without, and (b)with linking a composite probe (linking CK-19 antibody), power density of 808nm laser is 1.2 W/cm², radiating 10minutes. The result is shown in Fig 7. Probes prepared by nano flower of different sizes prepared in other examples have about the same shapes and effect, but the wavelength for generating photothermal effect is different, and being used for different applications.

### Example 5:

(1) 0.6 mL of 10 mM ice bathed NaBH₄ is added to 10mL of an aqueous solution that comprising 4.5 mM HAuCl₄ and 0.1M CTAB, the solution is aged for at least 3 hours at the temprature of 29°C. Small gold balls are generated.
(2) 6 mL of 0.5 mM HAuCl₄ solution, 6 mL of 0.2 M CTAC and 4.5 mL of 0.1 M AA are mixed and 0.3 mL of seed crystals prepared in step (1) is added. The solution is centrifugally dissolved in 1 mL of water to obtain larger gold balls.
(3) 5.8 mL DMF (including 0.43 mM PVP (MW=55000)) and 40 µL HAuCl₄ (24.28 mM) are injected into a small bottle, then 200 µL seed crystals prepared in step (2) is added, being stirred at the temprature of 80°C for 1 hour, and the seeds for growing gold nanoflower is obtained. The shape and size of the seed crystals are shown in Fig 8(e).
(4) 15 mL of 3 M PVP DMF solution is added with 180 µL HAuCl₄, and 960 µL of seed crystals prepared in step (3) is added, being stirred for several hours until the completion of the reaction. The generated gold nanoflower is centrifugally dissolved in an ethanol solution. The suggested excitation wavelength for generating a photo-thermal effect can be seen in the fifth curve from the left in Fig 3, which is 960 nm.
(5) The product of step (4) is added to a mixed solution of 40 mL ethanol, 8 mL water, and 0.8 mL ammonia with ultrasound assisted, then 1.6 mL of 1% TEOS ethanol solution is added, centrifugalizing for 12 hours, to generate a composition of gold nanoflower @silica; the surface of silica is functionally modified with 200 µL of 3-aminopropyl trimethoxysilane (APTMS), and then 2 mL of CdTeS QDs linked with carboxyl is added. Reacting for 24 hours, and then centrifugally dispersed in ethanol.
(6) Followed by the ethanol solution in step (5) added into the mixed solution of 20 mL ethanol, 4 mL water, and 0.8 mL ammonia, 400 microlitres of 1% TEOS ethanol solution is dropwise added, and reacting for 24 hours; and then 200 µL of MPTMS, APTMS, etc. are used for modifying the surface of the composite structure for having sulfydryl group and amino group on the surface. Then the probes can be transferred to a specified target position. In the present example, the composite probe with a size of 100 nm and the shape is consistent with the TEM of the gold nanoflower @QDs photothermal fluorescence composite probe in Fig 2, the only difference is the wavelength for generating photothermal effect, and is used for different applications. The temperature elevating curve (900nm excited light power density: 1.2 W/cm²) of the composite probe solution and the control group (an aqueous solution) are shown in Fig 5.

Although the examples of the present invention are exemplified, for an ordinary technician, the crafts used in these examples can be able to be modified, changed, and substituted without breaking away from the principle and spirit of the present invention. The scope of the present invention is limited by the attached claims and the equivalence.

## Claims

1. A gold nanoflower structure, **characterized in that**, is fabricated by using a gold octahedron or gold ball or gold tetrahedron as a seed, for obtaining a gold nano particle with round head columns arranged around its circumferential side, by reducing chloroauric acid with a weak reductant in a high concentration polyvinylpyrrolidone environment.

2. A gold nanoflower structure according to claim 1, **characterized in that** the diameter of the gold nanoflower is 45-150 nm, and column length is 10-30nm.

3. A method for preparing the gold nanoflower, comprising the following specific steps :
(1) obtaining a gold seed crystals solution by reducing chloroauric acid with NaBH₄, followed by growing a larger gold nano-ball with an assistant of reductant, and growing gold octahedron, gold ball or gold tetrahedron based on the gold nano-ball;
(2) using the gold octahedron, gold ball or gold tetrahedron obtained in step (1) as seed crystals, growing a gold nanoflower by reducing chloroauric acid with a weak reductant, in an environment of high concentration PVP; and thus obtaining chrysanthemum-like gold nanoflower with different sizes and column lengths by changing PVP concentration, amount of chloroauric acid, and amount and type of the gold seed crystals.

4. Based on the method according to claim 3, **characterized in that** mixing the solution of NaBH₄ and chloroauric acid further comprises an aging step, the aging temperature is between 27 °C and 32 °C and the aging time is more than 3 hours.

5. Based on the methods according to claim 3 or 4, **characterized in that** the molar ratio of the NaBH₄ to chloroauric acid in step (1) is 6-24:2-10.

6. Based on the method according to claim 3, **characterized in that** the reductant in step (1) is ascorbic acid.

7. Based on the method according to claim 3, **characterized in that** the environment of high-concentration PVP in step (2) refers to a polyvinylpyrrolidone solution with a concentration of 4-10 mM.

8. Based on the method according to claim 3, **characterized in that** the weak reductant in step (2) is N,N-dimethylformamide.

9. Based on the method according to claim 3, **characterized in that** the volume ratio of the chloroauric acid to gold seed crystals solution in step (2) is 1:1∼1:30.

10. A gold nanoflower/quantum dot composite probe for living cell immunofluorescent labeling and photothermal therapy, **characterized in** comprising the gold nanoflower structure according to claim 1 or 2.

11. Based on the composite probe according to claim 10, **characterized in** comprising, from inside to outside, the gold nanoflower, a silica nano shell layer, quantum dots and a silica nano shell layer; including wrapping a silica layer outside the gold nanoflower through Stöber hydrolysis based on the prepared gold nanoflower to form a silica ball wrapped with the gold nanoflower; then linking the quantum dots to the surface of the silica ball by modifying the surface of silica, to form a silica ball composite material, with the gold nanoflower wrapped inside and the quantum dots linked outside; and wrapping the surface of the composite material with another silica nano shell layer at the end.

12. Based on the method for preparing the gold nanoflower/quantum dot composite probe for living cell immunofluorescent labeling and photothermal therapyaccording to claim 10 or 11, including the following steps:
(1) preparing a gold nanoflower; and
(2) preparing gold nanoflower @silica @quantum dots composite material by:
centrifugally washing the gold nanoflower obtained in step (1), dissolving the nonoflower in a mixing liquid of ethyl ethanol, water and ammonia assistant with ultrasound, followed by dropwise adding an ethanol solution of tetraethyl orthosilicate to the reaction solution for wrapping the gold nanoflower with the silica ball shell, centrifugally washing after reacting for 12 hours, forming a composite structure of the gold nanoflower and the silica; and
surface modifying the above structure, adding aqueous solution of fluorescence quantum dots with carboxyl to the modified composite structure of gold nanoflower and silica, reacting for 24 hours, and then centrifugalizing and dispersing in ethanol, obtaining an ethanol solution of gold nanoflower @silica @quantum dots composite material;
(3) preparing a gold nanoflower @silica@quantum dot composite probe by:
adding ammonia to the composite material prepared in step (2) assistant with ultrasound, followed by dropwise adding an aqueous solution of tetraethyl orthosilicate, stirring at room temperature for reacting for 24 hours, centrifugal washing; modifying with various silane coupling agents to bring different modifying groups on surface thereof, and obtaining the composite probe at the end.

13. Biological targeting applications of the gold nanoflower/quantum dot composite probe for living cell immunofluorescent labeling and photothermal therapy according to claim 10 or 11.
